# Europäisches Patentamt

## European Patent Office

## Office européen des brevets

(19)

(11) Numéro de publication: **0 064 475**

**B1**

(12) # FASCICULE DE BREVET EUROPÉEN

(51) Int. Cl.⁴: **A 61 B 5/02**

(45) Date de publication du fascicule du brevet:
24.09.86

(21) Numéro de dépôt: **82810150.1**

(22) Date de dépôt: **06.04.82**

(54) Procédé de mesures de la pression sanguine d'un sujet et sphygmomanomètre utilisant ce procédé.

(30) Priorité: **24.04.81 DE 3116387**

(43) Date de publication de la demande:
**10.11.82 Bulletin 82/45**

(45) Mention de la délivrance du brevet:
**24.09.86 Bulletin 86/39**

(84) Etats contractants désignés:
**CH FR GB IT LI**

(56) Documents cité:
**DE-A-1 466 829**
**DE-A-2 555 453**
**US-A-3 552 381**
**US-A-4 058 117**

(73) Titulaire: **ASULAB S.A., Faubourg du Lac 6, CH-2502 Bienne (CH)**

(72) Inventeur: **Hatschek, Rudolf, Rue Vogt 3, CH- 3700 Fribourg (CH)**
Inventeur: **Bernau, Werner, Acacias 12, CH- 2000 Neuchatel (CH)**

(74) Mandataire: **Barbeaux, Bernard, SMH Société Suisse de Microélectronique et d'Horlogerie S.A. Département Brevets et Licences 6, Faubourg du Lac, CH- 2501 Bienne (CH)**

LIBER, STOCKHOLM 1986

## Description

La présente invention a pour objet un sphygmomanomètre, c'est-à-dire un appareil de mesure de la pression sanguine (ou tension artérielle) d'un sujet au moyen d'un brassard fixé sur un membre dudit sujet, ce brassard comportant une chambre déformable contenant un gaz sous pression.

Selon l'état actuel de la technique, on mesure la pression sanguine d'un sujet de la façon suivante:

on fixe sur le bras du sujet, le brassard muni de la chambre déformable qui est reliée à une pompe;

on fait croître la pression de gaz régnant dans la chambre jusqu'a une valeur de départ supérieure à la pression systolique attendue du sujet;

on fait décroître la pression régnant dans cette chambre depuis cette valeur de départ jusqu'à une valeur finale inférieur à la pression diastolique du sujet;

durant cette décroissance de pression, on détecte les sons émis dans une zone contenant ledit brassard, et on valide ceux dont l'intensité dépasse un certain seuil de validation, et

on mesure les pressions régnant dans la chambre lors, respectivement, de la première et de la dernière validation d'un son.

La pression mesurèe lors de la première validation d'un son est la pression systolique et celle qui est mesurée lors de la dernière validation d'un son est la pression diastolique.

Le brevet américain No 3,450,131, par exemple, décrit un sphygmomanomètre comportant un microphone pour la détection des sons émis par l'artère du sujet examiné, ces sons étant communément appelés sons de Korotkoff. Ce microphone est relié par l'intermédiaire d'un amplificateur aux entrées de trois filtres passe-bande dont les sorties sont chacune reliées par l'intermédiaire d'un formateur d'impulsions à une bascule ou trigger de Schmitt. Ces bascules produisent des impulsions rectangulaires pendant les intervalles de temps durant lesquels les signaux qu'ils reçoivent dépassent un seuil prédéterminé. Ces impulsions sont ensuite appliquées à un circuit logique en vue de commander l'enregistrement de la pression.

Pour effectuer une mesure de la tension artérielle, on gonfle la chambre déformable du brassard à une pression supérieure à la pression systolique attendue du sujet, puis on relâche lentement la pression. Les sons de Korotkoff produits dans un certain domaine de pression sont transformés par le microphone en signaux électriques. Le circuit logique aux sorties des bascules de Schmitt est conçu de telle façon que les signaux qui présentent une composante de 1000 Hz sont identifiés comme son parasite, tandis que les signaux présentant une composante de 40 Hz et une composante de 100 Hz, mais pas de composante de 1000 Hz, sont identifiés comme sons de Korotkoff. Pour chaque signal identifié comme son de Korotkoff, la valeur momentanée de la pression régnant dans la chambre déformable, valeur mesurée par un capteur de pression, est enregistrée dans un appareil enregistreur de pression. La première valeur enregistrée correspond alors à la pression systolique et la dernière valeur enregistrée correspond à la pression diastolique.

Ainsi, dans ce sphygmomanomètre connu, la mesure des pressions systolique et diastolique est faite indépendemment des caractéristiques individuelles du sujet, et en particulier indépendamment de la valeur absolue de ces pressions. Si l'on utilise des bascules de Schmitt dont les seuils sont réglés à des valeurs correspondant à une intensité sonore moyenne de son de Korotkoff, évaluée avec un grand nombre de sujets, il peut arriver que pour certains sujets, les sons de Korotkoff soient si faibles que les signaux transmis aux bascules de Schmitt n'atteignent plus, au moins en partie, lesdites valeurs de seuil. Cela conduit à des erreurs de mesure considérables ou peut même rendre impossible une mesure de la tension artérielle. Si, au contraire, on utilise des bascules de Schmitt ayant des seuils suffisamment bas pour permettre la détection de sons de Korotkoff extrêmement faibles, la distinction entre les signaux de sons de Korotkoff et les signaux de sons parasites serait très difficile dans le cas ou l'on aurait des suites de sons de Korotkoff ayant une entensité sonore moyenne ou grande, et cela intraînerait de nombreuses erreurs de mesures.

C'est pourquoi la présente invention a notamment pour objet de proposer un procédé de mesure de pression sanguine qui permette de détecter aussi bien les suites de sons de Korotkoff de faibles intensés que celles qui sont composées de sons de Korotkoff de fortes intensités, sans présenter une sensibilité aux sons parasites.

Selon l'invention, le seuil de validation des sons est ajusté en dépendance, de façon croissante, de la pression régnant dans la chambre déformable du brassard.

Ainsi, l'invention est basée principalement sur l'utilisation de la connaissance obtenue par des expériences et des recherches, à savoir qu'une partie essentielle des erreurs de mesure des appareils de mesure de pression sanguine connus est due aux différences, existant entre les individus, de l'intensité sonore des sons de Korotkoff, et que l'intensité sonore des suites de sons de Korotkoff et d'au moins d'une partie des sons parasites, c'est-à-dire des sons qui ne sont pas dus au passage du sang dans l'artère du sujet, présente une forte corrélation avec la tension artérielle du sujet.

Certes, il est connu, notamment par le document allemand No 1 466 829, d'ajuster le seuil de validation des sons en fonction de la pression régnant dans la chambre déformable du brassard. Toutefois, alors que, selon l'invention, cet ajustement est réalisé en dépendance de façon croissante de la pression, le seuil utilisé, dans ce document, pour la détermination de la pression diastolique (la plus basse) est plus élevé que le seuil utilisé pour la détermination de la pression systolique. Grâce à une telle disposition, la pression systolique peut être prise à l'apparition des sons de Korotkoff et la pression diastolique, non pas à la disparition de ceux-ci, mais au moment où leur intensité commence à s'affaiblir.

Les pressions systolique et diastolique mesurées avec un appareil selon l'invention ont été comparées avec les valeurs obtenues par des médecins expérimentés utilisant la méthode classique et se servant d'un stéthoscope. Les mesures ont porté sur environ 500 personnes, parmi lesquelles se trouvaient des patients ayant une tension artérielle anormale, en particulier excessive ou trop faible. On obtient une concordance pratiquement parfaite entre les mesures effectuées selon les deux procédés de mesure.

Mentionnons encore pour plus de clarté que dans les revendications ou le reste de la description, les expressions "pression sanguine" et "pression dans la chambre gonflable" se rapportent toujours à la surpression par rapport à la pression athmosphérique ambiante.

Les dessins annexés représentent, à titre d'exemple, une forme de réalisation de l'objet de l'invention.

La fig. 1 est une vue de dessus d'un sphygmomanomètre selon un mode de réalisation de l'invention.

La fig. 2 est un schéma synoptique du sphygmomanomètre de la figure 1.

La fig. 3 est un schéma des éléments de circuit électronique les les plus importants servant à fixer la valeur de seuil variable.

La fig. 4 est un diagramme illustrant la variation de la pression au cours du temps, lors d'une mesure.

La fig. 5 est un diagramme illustrant la variation au cours du temps de la valeur de seuil et des signaux de sons de Korotkoff lors d'une mesure.

La fig. 6 est un diagramme correspondant à la fig. 5, mais lors d'une mesure sur du sujet à tension artérielle élevée.

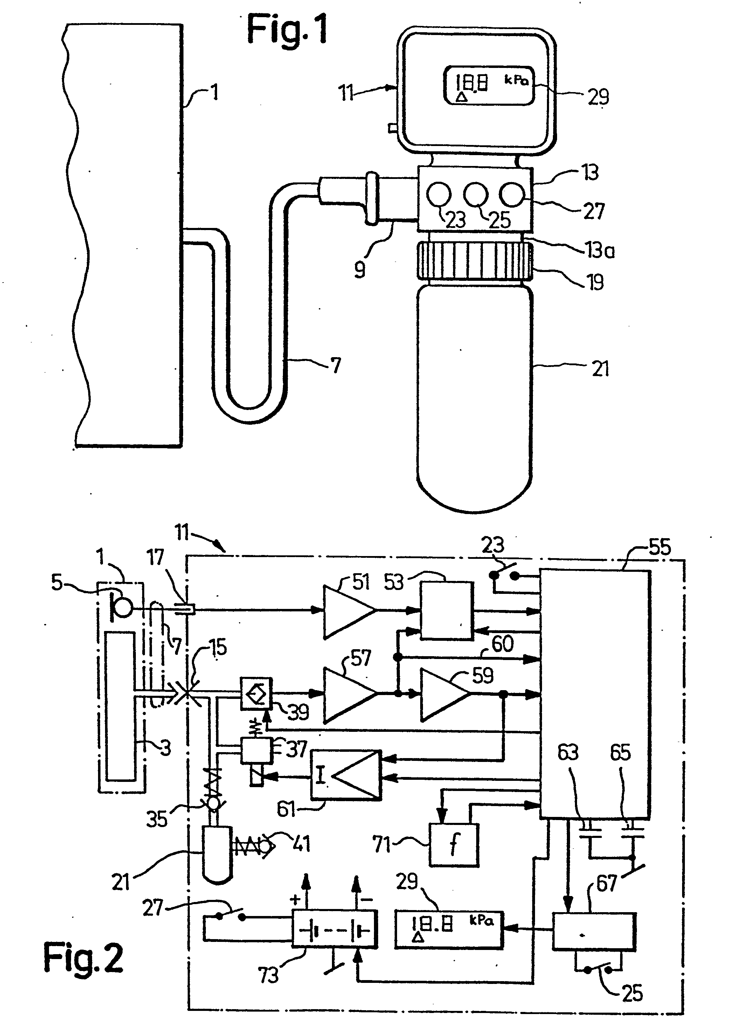

Le sphygmomanomètre représenté aux fig. 1 et 2 comprend un brassard 1 pouvant être fixée au bras du sujet. Le brassard est muni, d'une part, d'une chambre d'air 3 étanche, déformable, formée d'une poche en caoutchouc, et, d'autre part, d'un microphone 5. Un conduit 7 est relié à demeure par une extrémité au brassard 1 ; son autre extrémité est reliée de façon amovible par un accouplement à fiche 9, à un appareil 11. Ce dernier présente un boîtier rigide 13 sur lequel sont fixées une prise d'air 15 et une connexion électrique 17 (voir fig. 2). La chambre 3 est reliée par un tuyau 3a disposé dans le conduit 7, avec la prise d'air 15, et le microphone 5 est relié par un câble situé dans le conduit 7, avec la connexion électrique 17. Le boîtier 13 est muni d'un manchon fileté 13a sùr lequel est fixée de façon amovible une pompe 21 formée par un corps creux sensiblement cylindrique, en caoutchouc. L'appareil présente en outre trois boutons-poussoirs 23, 25, 27, une unité d'affichage numérique 29 et divers éléments pneumatiques et électroniques, disposés à l'intérieur du boîtier 13.

La chambre 3 est reliée par le tuyau 3a et par des passages d'air situés dans l'appareil 11, par l'intermédiaire d'une soupape anti-retour 35, à la pompe 21, ainsi qu'à une valve de décompression 37 actionnable électriquement et à un capteur de pression 39. La valve 37 est du type modérable, c'est-à-dire qu'elle présente un étranglement ou produit une perte de charge variable de façon continue et dont la valeur dépend de l'amplitude d'un signal de commande appliqué sur ladjte valve. La pompe 21 est encore munie d'une entrée d'air présentant une soupape anti-retour 41. Les deux soupapes anti-retour 35 et 41 sont montées de telle façon qu'en comprimant et en relâchant alternativement, à la main, le corps creux de la pompe 21, on prélève de l'air dans l'environnement et on gonfle la chambre 3.

Le microphone 5 est relié électriquement à l'entrée d'un amplificateur 51 dont la sortie est reliée à l'entrée d'un discriminateur 53. La sortie du discriminateur 53 est reliée à un dispositif de pilotage 55.

Le capteur de pression 39 fournit une variable électrique donnant une mesure pour la pression régnant dans la chambre 3; ce capteur contient un montage en pont servant de transformateur de mesure, formé d'éléments piézorésistifs, et il est relié à l'entrée d'un amplificateur 57 et, par l'intermédiaire d'un circuit différentiateur 59 et d'une liaison 60 en parallèle à ce dernier, au dispositif de pilotage 55. La sortie du circuit différentiateur 59 est également reliée à une entrée d'un régulateur 61. Le dispositif de pilotage 55 est relié à une autre entrée du régulateur 61, dont la sortie est reliée à l'organe de commande électromagnétique de la soupape de décomression 37.

Le dispositif de pilotage 55 présente deux bornes qui sont respectivement reliées à des condensateurs formant des mémoires analogiques 63 et 65. Le dispositif de pilotage 55 est relié en outre à un circuit de commande d'affichage 67 contenant entre autres un convertisseur analogique-numérique. Le circuit 67 est lui-même relié à l'unité d'affichage 29. Le dispositif de pilotage 55 est aussi relié à une entrée d'un cardiotachymétre 71 comportant une mémoire. Le bouton-poussoir 23 est relié au dispositif de pilotage 55 et le bouton-poussoir 25 au circuit 67. Il est prévu en outre une source d'alimentation électrique 73 contenant une batterie, qui est reliée aux bornes d'alimentation des différents éléments actifs de l'appareil 11 et à la masse.

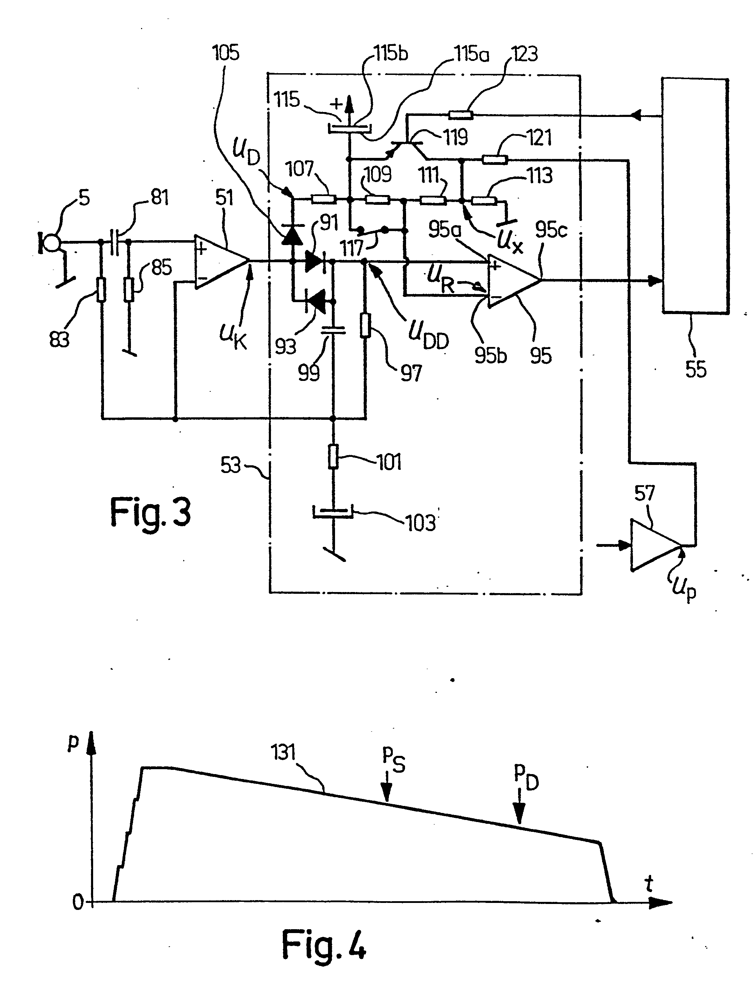

On va expliquer maintenaǹt le schéma de la fig. 3, représentant le discriminateur 53 et quelques composants électroniques reliés à celuici.

Le microphone 5 est relié à l'amplificateur 51 par l'intermédiaire d'un condensateur 81 ; celui-ci est relié à l'entrée non inverseuse de l'amplificateur 51 et, par l'intermédiaire d'une résistance 85, à la masse électrique. En outre, le microphone 5 est relié, par l'intermédiaire d'une résistance 83, et l'entrée inverseuse de l'amplificateur 51, directement, à des éléments constitutifs du discriminateur 53.

La sortie de l'amplificateur 51 est reliée à l'entrée non inverseuse 95a d'un amplificateur différentiel 95, par l'intermédiaire de deux diodes 91 et 93 appartenant au discriminateur 53, ces diodes étant montées en parallèle et présentant des sens de conduction opposés. L'entrée 95a est en outre reliée, par l'intermédiaire d'une résistance 97 et d'un condensateur 99 monté en parallèle avec elle, d'une part, à la jonction entre l'entrée inverseuse de l'amplificateur 51 et la résistance 83 et, d'autre part, par l'intermédiaire d'une résistance 101 et d'un condensateur 103 montés en série avec elle, à la masse. La sortie de l'amplificateur 51 est encore reliée à la masse par l'intermédiaire d'une troisième diode 105 et de quatre résistances 107, 109, 111 et 113 montées en série avec elle et entre elles. Le point de jonction entre les résistances 107 et 109 est relié à une électrode 115a d'un condensateur 115 dont l'autre électrode 115b est reliée au pôle positif de la source d'alimentation.

Un interrupteur 117 actionnable à la main, est monté en parallèle avec la résistance 109. Cet interrupteur est logé dans un compartiment du boîtier 13 et il est accessible lorsque le couvercle ferment normalement ce compartiment est ouvert ou enlevé.

Le point de jonction entre les résistances 109 et 111 est relieé a l'entrée inverseuse 95b de l'amplificateur 95. Un transistor de commutation 119 présente un émetteur relié à l'électrode 115a du condensateur 115 et un collecteur qui est relié aussi bien au point de jonction entre les résistances 111 et 113 que, par l'intermédiaire d'une résistance 121, à la sortie de l'amplificateur 57. La base du transistor 119 est reliée, par l'intermédiaire d'une résistance 123, à une sortie du dispositif de pilotage 55. La sortie 95c de l'amplificateur 95 est reliée à une entrée du dispositif de pilotage 55.

Les condensateurs 81, 99, 103 et 115 peuvent respectivement avoir par exemple des capacités de 3,3μF, 10μF, 4,7μF et 3,3μF. Les résistances peuvent avoir par exemple les valeurs suivantes:
Résistance 83 470 kΩ, résistance 85 2,2 MΩ;
Résistance 97 100 kΩ, résistance 101 1 kΩ;
Résistance 107 4,7 kΩ, résistance 109 8,8 MΩ;
Résistance 111 3,3 MΩ, résistance 113 2,2 kΩ;
Résistance 121 10 kΩ, résistance 123 22 MΩ.

Quand au trois diodes 91, 93 et 105, il s'agit de diodes au silicium identiques, par exemple des diodes du type 1 N 4148. Comme transistor de commutation 119, on peut prendre par exemple le transistor BC 214.

Le dispositif de pilotage 55 est formé essentiellement d'un circuit intégré présentant un certain nombre de bascules et de portes servant à effdctuer des opérations logiques. Le dispositif de pilotage 55 comprend en outre des éléments raccordés au circuit intégré, en particulier des condensateurs pour fixer divers intervalles de temps.

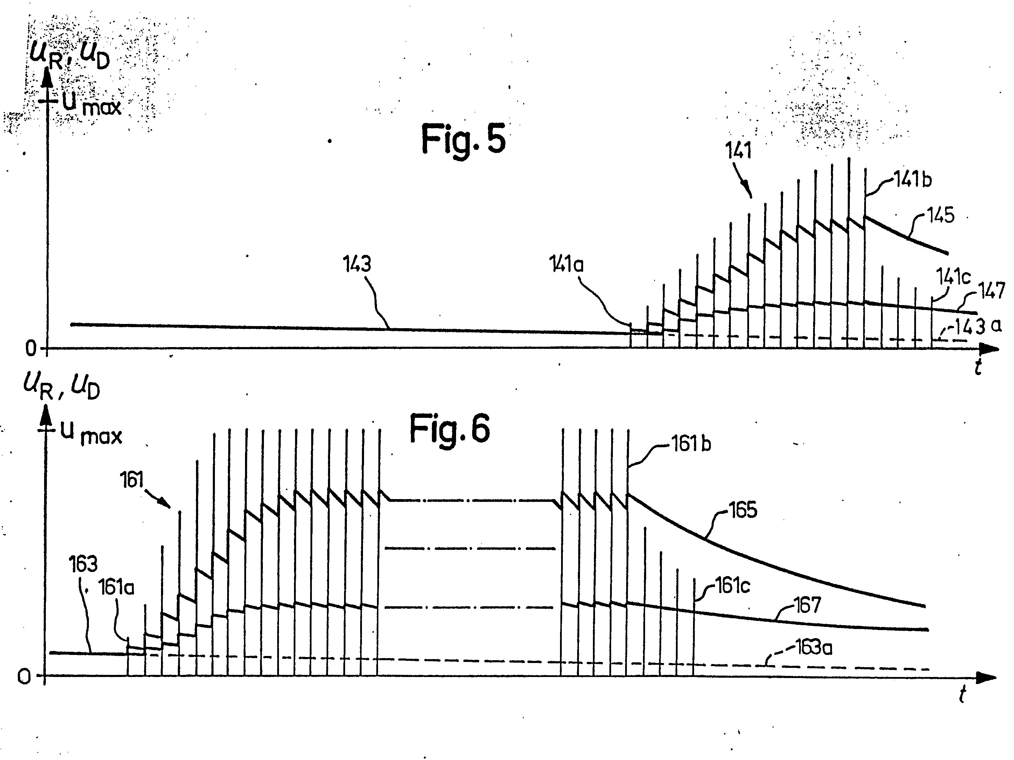

On va expliquer maintenant le fonctionnement du sphygmomanomètre qui vient d'être décrit. Tout d'abord, on décrira le principe général de mesure, en regard de la fig. 4, dans laquelle la courbe 131 représente l'évolution, au cours d'une mesure, de la pression p dans la chambre 3 en fonction du temps t. Ensuite, on expliquera encore quelques détails en regard des fig. 5 et 6.

Pour mesurer la tension artérielle, on fixe le brassard 1 à un membre, en général un bras, d'un sujet. L'appareil 11 auquel le brassard 1 est relié, est ensuite mis en service en exerçant une courte pression sur le bouton-poussoir 27. Dans le laps de temps qui suit, pendant lequel la valve 37 est entièrement ouverte et la chambre 3 sans pression, le capteur de pression 39 est mis à zéro automatiquement par un dispositif de recalage à zéro non représenté. Ensuite, en actionnant manuellement la pompe 21, on gonfle par à-coups la chambre 3, de sorte que la pression p régnant dans cette chambre augmente. La soupape 37 est fermée automatiquement par le dispositif de pilotage 55 pendant le gonflage.

La valve 37 reste encore fermée, après le dernier coup de pompe, pendant un laps de temps prédéterminé, par exemple de 1 à 2 secondes, cette valve est ensuite ouverte par le dispositif de pilotage 55 et son degré d'ouverture est réglé par le régulateur 61 de telle façon que la pression p décroisse de façon linéaire, c'est-à-dire à une vitesse constante par exemple égale 300 à 500 Pa/s.

Pendant une certaine partie de la chute de pression linéaire, le déplacement du sang dans l'artère comprimée par le brassard 1, produit une suite de sons de Korotkoff. Des signaux électriques de sons de Korotkoff $U_K$ sont ainsi formés et sont comparés dans le discriminateur 53 avec une valeur de seuil variable $U_R$.

Lorsque l'amplitude d'un signal de son de Korotkoff $U_K$ dépasse ladite valeur de seuil, le discriminateur 53 envoie un signal de validation au dispositif de pilotage 55. Ce dernier réagit à l'apparition du premier signal de validation de façon que la valeur momantanée de la pression p soit détectée comme pression systolique $P_S$ et soit stockée dans le condensateur 63 servant de mémoire. Le dispositif de pilotage 55 réagit en outre à chaque signal de validation ultérieur de façon que la valeur momentanée de la pression p soit détectée et soit stockée dans le condensateur 65 servant de mémoire. Après la détection et le stockage de la dernière valeur de la pression, qui constitue la pression diastolique $P_D$, le dispositif de pilotage agit après un laps de temps prédéterminé de durée comprise, par exemple, entre 3 et 6 secondes, de façon que la valve 37 s'ouvre complètement et que la pression p retombe rapidement à zéro.

On va expliquer maintenant plus en détail la formation de la valeur de seuil $U_R$. Pour chaque son de Korotkoff, à l'exception du premier son validé par le discriminateur 53, la valeur de seuil $U_R$ dépend non seulement de la pression p régnant dans la chambre 3, mais aussi de l'intensité sonore des sons de Korotkoff, comme cela est expliqué ci-dessus.

Les signaux électriques basse fréquence produits par le microphone 5 lors de l'apparition des sons de Korotkoff sont amplifiés par l'amplificateur 51. Ce dernier constitue aussi, en liaison avec les résistances et les condensateurs associés, un filtre passe-bande. A la sortie de l'amplificateur 51 apparaît, par rapport à la masse électrique, pour chaque son de Korotkoff, un signal électrique ($U_K$) constitué par un paquet d'ondes, c'est-à-dire une tension alternative $U_K$ alternant par rapport à la masse, et apparaissant pendant un certain laps de temps. La diode 105 agissant comme redresseur ne laisse passer que les demi-ondes positives par rapport à la masse, de la tension alternative $U_K$, demi-ondes qui sont ainsi transmises au circuit RC fourni par les résistances 107, 109, 111 et 113 et par le condensateur 115. A la cathode de la diode 105 apparait donc pour chaque son de Korotkoff la moitié positive d'un paquet d'ondes. Cette tension continue pulsée sera désignée par $U_D$. Sur les fig. 5 et 6 on a représenté, pour chaque mesure, une suite respectivement 141 et 161 de signaux de sons de Korotkoff transmis par la diode 105, s'étendant sur un certain intervalle de temps t. A chaque son de Korotkoff est associé un trait dont la hauteur est égale à la valeur de crête, c'est-à-dire à la plus grande amplitude, des demi-ondes traversant la diode 105 lors de l'émission du son de Korotkoff correspondant.

Les deux diodes 91 et 93 montées en parallèle et présentant des sens de conduction opposés n'opèrent pas de redressement, mais elles ont pour but de modifier la tension alternative $U_{DD}$ transmise par elles à l'entrée 95a de l'amplificateur 95 de telle façon que ses demi-ondes positives par rapport à la masse coïncident le mieux possible, quant à l'amplitude et à la forme, avec les signaux traversant la diode 105. L'amplificateur 95 est une bascule- ou trigger- de Schmitt servant de comparateur de tension et fournissant, chaque fois que la tension alternative $U_{DD}$ atteint ou dépasse la valeur de seuil $U_R$, une impulsion sensiblement rectangulaire. Lors de chaque son de Korotkoff pour lequel la valeur de crête de $U_{DD}$ et donc aussi celle de $U_D$, atteint au moins la grandeur seuil $U_R$, l'amplificateur 95 transmet un signal de validation constitué le plus souvent par un paquet d'impulsions, au dispositif de pilotage 55.

Sur les fig. 5 et 6 on a représenté, en outre, des parties de courbe 143, 145, 147, respectivement 163, 165, 167, représentant la variation au cours du temps d'une tension continue, mesurée par rapport à la masse et transmise à l'entrée 95b de l'amplificateur 95, qui représente la valeur de seuil $U_R$.

Comme cela a été dit plus haut, la chambre 3 subit une décompression durant une phase commençant peu après la fin du pompage, cette décompression étant effectuée de telle façon que la pression p diminue linéairement avec le temps. Par conséquent, la tension continue $U_p$ recueillie à la sortie de l'amplificateur 57, étant proportionnelle à la pression p, elle diminue linéairement avec le temps. Aussi longtmeps qu'aucun signal n'apparaît à la sortie de l'amplificateur 51 dans la partie initiale de la phase de décompression, la tension $U_x$ présente au point de jonction des trois résistances 111, 113 et 121 est proportionnelle à la pression p avec une très bonne approximation. La tension représentant la valeur de seuil $U_R$ dépend très peu de la position de l'interrupteur 117 pendant cette partie de la phase de décompression; cette tension est en particulier proportionnelle à la pression p avec une bonne approximation pour les deux positions de l'interrupteur 117. Pour les parties de courbes droites 143 et 163 et leurs prolongements 143a et 163a représentés en pointillé sur les figures 5 et 6, on a donc avec une bonne approximation, les relations suivantes:

$$U_x = c_x p \quad (1)$$
$$U_R = c_R p \quad (2)$$

où $c_x$ et $c_R$ sont des constantes de grandeurs semblables et approximativement égales pour les deux positions de l'interrupteur 117.

La tension transmise à l'entrée 95b de l'amplificateur 95, représentant la valeur de seuil $U_R$, est donc une fonction linéaire de la pression p; elle est même proportionnelle à la pression p, et donc décroît linéairement avec le temps avant l'apparition des signaux de sons $U_K$.

Lorsque des signaux ($U_K$) représentant les sons de Korotkoff ou éventuellement des parasites, apparaissent à sortie de l'amplificateur 51, ils n'influencent la valeur de seuil $U_R$, que si leur amplitude crête dépasse la courbe 143, respectivement 163. Si les amplitudes des signaux apparaissant à la sortie de l'amplificateur 51 ne dépassent pas la courbe 143, respectivement 163, la diode 105 bloque la liaison conduisant de la sortie de l'amplificateur 51 au circuit RC formé par les résistances 107, 109, 111, 113, 121 et le condensateur 115. La valeur de seuil $U_R$ continue donc de dépendre uniquement de la pression p.

Dès qu'à la sortie de l'amplificateur 51 apparaît un signal ($U_K$) ci-après, qualifié d'actif, dont l'amplitude crête dépasse la grandeur momentanée de la valeur de seuil $U_R$, la diode 105 devient temporairement conductrice. Dans ledit circuit RC il se produit alors une superposition - ou un mélange - de la tension continue U délivrée par l'amplificateur 57, proportionnelle à la pression p, et de la tension pulsée $U_D$.

Dans ce qui suit, pour autant qu'on ne dise pas le contraire, on admet que le transistor de commutation 119 se trouve dans son état bloqué. Par conséquent, l'organe de commutation électronique formé par le transistor 119 est ouvert.

Les résistances 113 et 121 sont sensiblement plus petites, à savoir environ 100 à 10'000 fois plus petites, que les résistances 109 et 111. La tension $U_x$ au point de jonction des trois résistances 111, 113, 121 est, par conséquent, pratiquement indépendante de la grandeur de la tension $U_K$ et donc, aussi, dépendante presque exclusivement de la pression p lors de l'apparition de hauts signaux de sons de Korotkoff. La relation (1) est donc valable avec une bonne approximation également lors de l'apparition de signaux de sons dépassant la valeur de seuil et donc aussi la tension $U_x$. Cette dernière tension a donc approximativement l'allure représentée par les prolongements 143a et 163a dessinés en pointillés, des parties de courbes droites 143, 163.

A l'apparition de chaque son de Korotkoff pour lequel la valeur crête du signal $(U_K)$ dépasse la grandeur de la valeur de seuil $U_R$, cette valeur de seuil augmente brusquement en tendant vers une valeur limite supérieure qui est une fonction croissante de ladite valeur crête, puis dès que la tension $U_K$ devient inférieure au seuil $U_R$, ce seuil $U_R$ décroît exponentiellement. Cette allure dans le temps de la valeur de seuil est représentée sur les fig. 5 et 6 par les parties de courbe 145, 147, 165, 167. Les courbes 145, 165 sont valables pour le cas où l'interrupteur 117 est fermé, comme représenté dans la fig. 3. Si au contraire, l'interrupteur 117 est ouvert, la valeur de seuil a l'allure représentée par les parties de courbes 147, 167.

Lors de la chute exponentielle de la valeur de seuil après une élévation brusque, cette valeur de seuil tend vers une valeur-limite inférieure U qui est variable avec le temps et dont l'allure est donnée par les prolongements 143a 163a dessinées en pointillé des parties de courbes droites 143, 163; cette valeur-limite est donc proportionnelle à la pression momentanée p.

Ainsi, on peut dire que la valeur du seuil $U_R$ permettant de valider un signal de son courant $U_K$ dépend, selon une fonction croissante, de l'amplitude crête d'au moins le signal de son précédemment validé immédiatement avant. On conçoit cependant que la valeur de seuil $U_R$ peut, dans un moindre mesure, dépendre également, selon une fonction croissante, de l'amplitude crête du signal de son validé immédatement avant ledit signal précédant.

La constante de temps avec laquelle la valeur de seuil croît lors des élévations brusques est approximativement indépendante de la position de l'interrupteur 117 et elle est égale au plus à 100 millisecondes; cette valeur est par exemple comprise entre 5 et 30 millisecondes. Par contre, la constante de temps avec laquelle la valeur de seuil décroît après les élévations brusques dépend fortement de la position de l'interrupteur 117. Si l'interrupteur 117 est fermé la constante de temps lors de la décroissance de la valeur de seuil est typiquement comprise entre 15 et 5 secondes; cette valeur est par exemple de 10 secondes. Si l'interrupteur 117 est ouvert, ladite constante de temps est typiquement comprise entre 50 et 20 secondes; elle est, par exemple, de 30 secondes. La constante de temps lors de la décroissance de la valeur de seuil $U_R$ est donc égale, lorsque l'interrupteur 117 est fermé, à environ 10 à 75% et de préférence à 40 à 60% de la constante de temps existant pour l'interrupteur ouvert.

Les accroissements de la valeur de seuil qui ont lieu lors des élévations brusques de la valeur de seuil sont aussi dépendants de la position de l'interrupteur 117. Si l'interrupteur 117 est fermé, la valeur de seuil se rapproche davantage, lors des élévations brusques, des valeurs de crête des signaux de son de Korotkoff $(U_K)$ que si l'interrupteur est ouvert. Comme, lors des élévations brusques, le condensateur 115 doit être chargé par l'intermédiaire de la diode 105 et de la résistance 107, il se produit, lors de ces élévations une certaine intégration et un certain nivellement.

Lorsque quelques signaux de son successifs dépassant la valeur de seuil $U_R$ ont une grandeur constante, comme c'est le cas dans la partie centrale de la suite des signaux de sons de Korotkoff 161, la valeur de seuil $U_R$ tend vers une valeur limite supérieure. Cette dernière est, au moins approximativement, proportionnelle à l'amplitude des signaux de son de Korotkoff $(U_K)$. Si l'interrupteur 117 est fermé, la valeur limite est égale à environ 50 à 90% et, par exemple, à environ 75% de la valeur de crête du signal de son $(U_K)$ correspondant. Si l'interrupteur 117 est ouvert, ladite valeur-limite est égale à environ 20 à 40%, par exemple à environ 30% des valeurs de crête des signaux de son de Korotkoff $(U_K)$.

Comme cela a déjà été dit, la pression systolique est mesurée au moment où se produit le premier signal de son de Korotkoff atteignant au moins la grandeur de la valeur de seuil $U_R$. Ce premier signal est, indépendamment de la position de l'interrupteur 117, le signal 141a de la suite 141, respectivement le signal 161a de la suite 161. Les signaux 141b et 161b correspondent à ce qu'on appelle le quatrième son de Korotkoff, c'est-à-dire au son après lequel l'intensité sonore décroît fortement. Si l'interrupteur 117 est ouvert, la pression diastolique est mesurée lors de l'apparition du signal du son de Korotkoff 141c, respectivement 161c. Ces signaux 141c, 161c correspondent à ce qu'on appelle le cinquième son de Korotkoff, qui est sensiblement plus faible et aussi plus grave que le quatrième son de Korotkoff. Si l'interrupteur 117 est ouvert, on mesure donc, toutes conditions étant égales par ailleurs, une plus petite pression diastolique que si l'interrupteur 117 est fermé.

Comme cela a déjà été mentionné plus haut, l'intensité sonore maximale des sons de Korotkoff peut varier fortement d'un sujet à l'autre, les maxima de l'intensité sonore variant dans un rapport dix ou davantage. L'intensité sonore dépend fortement de la pression sanguine de la personne examinée. Chez les personnes ayant une pression sanguine élevée, l'intensité sonore est en général considérablement plus grande que chez les personnes ayant une pression sanguine moyenne ou même très faible.

Les diagrammes des fig. 5 et 6 sont positionnés les uns par rapport aux autres de manière que des valeurs identiques du temps t correspondent à la méme pression p. Dans le diagramme de la fig. 6, la suite de sons de Korotkoff 161 se trouve dans un domaine de pressions comprenant des pressions plus élevées que dans le cas de la suite 141 représentée dans le diagramme de la fig. 5. Par conséquent, les signaux de son de Korotkoff de la suite 161 ont aussi en général des amplitude plus élevées que pour la suite 141. La partie proportionnelle à la pression p de la valeur de seuil $U_R$, qui est représentée par la partie de courbe droite 143, respectivement 163, est plus élevée pour le signal de son 161a, pour lequel on mesure la pression systolique, que pour le signal 141a de la suite 141.

Du fait que la valeur de seuil est modifiée de la manière décrite, on obtient que cette valeur de seuil, lors de la mesure de la pression systolique, soit plus grande pour une forte intensité sonore des sons de Korotkoff ultérieurs que pour une faible intensité sonore de ces sons. Egalement, lors de la mesure de la pression diastolique, la valeur de seuil $U_R$ est plus grande pour une grande intensité sonore des sons de Korotkoff que pour une faible intensité sonore de ces sons. Cela permet d'effectuer des mesures de la pression sanguine avec une bonne exactitude même chez des personnes chez lesquelles les pressions sanguines sont extrêmement hautes ou extrêmement basses et chez lesquelles, par conséquent, les sons de Korotkoff sont extrêmement forts ou extrêment faibles.

Comme cela ressort de la description qui précède, on sélectionne, au moyen de l'interrupteur 117 le mode de mesure requis, parmi les deux modes selon lesquels la pression diastolique doit être mesurée lors du quatrième, respectivement du cinquième son de Korotkoff. Si donc, à titre d'exemple, le sphygmomanomètre est utilisé par un patient souffrant d'une anomalie de la pression sanguine pour un contrôle personnel de sa pression sanguine, le médecin qui soigne ce patient a la possibilité de décider, en se basant sur des mesures de comparaison effectuées au moyen d'un stéthoscope, si le patient doit mesurer sa pression diastolique lors du quatrième ou du cinquième son de Korotkoff.

Le dispositif de pilotage 55 réagit au signal fourni par le différentiateur 59 afin de ne pas tenir compte des signaux de son ($U_K$) produits pendant le pompage et un intervalle de temps ultérieur de durée prédéterminée, par exemple de 1 à 3 secondes, de sorte que les signaux se produisant dans cet intervalle de temps ne peuvent pas provoquer de mise en mémoire de la pression p. Le dispositif de pilotage 55 est en outre agencé de telle manière que, pendant le pompage et ledit intervalle de temps ultérieur, il ferme l'organe de commutation électronique formé par le transistor 119. Les résistances 109 et 111 sont alors court-circuitées et les deux résistances 107 et 113 forment pour les signaux venant de l'amplificateur 51 un diviseur de tension.

Si l'on faisait tout d'abord abstraction du condensateur 115, ce diviseur de tension reduirait la tension pulsée venant de la diode 105 de telle façon que la tension présente sur l'émetteur du transistor 119 soit égale à au plus 50%, par exemple à environ 30% de la tension pulsée présente au point de jonction entre la diode 105 et la résistance 107. Si maintenant on tient compte de l'action du condensateur 115, celui-ci lorsque le transistor 119 se trouve à l'état conducteur, se décharge trés vite, avec une constante de temps d'au plus 30 et par exemple, de 5 à 10 millisecondes. Lorsque l'organe de commutation formé par le transistor 119 est fermé, les signaux parasites apparaissant à la sortie de l'amplificateur 51 ne modifient pratiquement pas la valeur de seuil $U_R$, même s'ils dépassent ce dernier.

Le sphygmomanomètre qui vient d'être décrit peut être modifié de différentes manières. A titre d'exemple, la valeur-limite inférieure $U_x$ qui dépend de la pression p, peut ne pas être proportionnelle à la pression. Cette valeur-limite pourrait, en effet, être liée d'une autre façon à la pression p, de manière à décroître lorsque cette pression baisse. Cette valeur-limite pourrait par exemple décroître par gradins lorsque la pression p diminue.

## Revendications

1. Sphygmomanomètre comprenant:
- un brassard (1) destiné à être fixé à un membre d'un sujet à examiner, ce brassard comportant une chambre déformable (3);
- des moyens de compression (21, 35, 41) pour augmenter la pression de gaz (p) régnant dans ladite chambre;
- des moyens de décompression (37) pour diminuer ladite pression;
- des moyens de mesure de pression (39) réagissant à ladite pression en fournissant un signal de pression (U) représentatif de cette pression;
- des moyens de détection de son (5, 51) réagissant à chaque son émis dans une zone contenant ledit brassard, en fournissant un signal ($U_K$) représentatif dudit son;
- des moyens (105, 107, 109, 111, 113, 115, 117, 119, 121, 123) pour déterminer une valeur de seuil ($U_R$), qui répondent au signal de pression ($U_p$) en ajustant ledit seuil en fonction de l'amplitude dudit signal de pression;
- des moyens de validation (53) répondant à ladite valeur de seuil ainsi qu'aux signaux représentatifs de son ($U_K$) dont la valeur sépasse ledit seuil ($U_R$), en fournissant un signal de validation; et
- des moyens de pilotage (55, 61, 63, 65) répondant auxdits signaux de pression ($U_p$) et de validation, en mesurant et en mettant en mémoire

13 **0 064 475** 14

les amplitudes que présente ledit signal de pression ($U_p$) lors de l'apparition, respectivement, du premier et du dernier signal de validation; caractérisé en ce que lesdits moyens de détermination de seuil ajustent ledit seuil ($U_R$) en dépendance, de façon croissante, de l'amplitude du signal de pression ($U_p$).

2. Sphygmomanomètre selon la revendication 1, caractérisé en ce que lesdits moyens de détermination de seuil répondent en outre à chaque signal représentatif de son actif ($U_K$) dont la valeur dépasse la valeur instantanée dudit seuil en réglant ledit seuil en dépendance, de façon croissante, de la valeur de ce signal de son.

3. Sphygmomanomètre selon la revendication 2, caractérisé en ce que lesdits moyens de détermination de seuil répondent à l'application dudit signal représentatif de son actif ($U_K$) en augmentant la valeur dudit seuil en la faisant tendre, suivant une première constante de temps, vers une première valeur-limite et à la disparition dudit signal en diminuant cette valeur de seuil en la faisant tendre, suivant une seconde constante de temps inférieur à la première constante de temps, vers une seconde valeur-limite.

4. Sphygmomanomètre selon la revendication 3, caractérisé en ce que ladite première valeur-limite dépend, de façon croissante, toutes choses étant égales par ailleurs, de la valeur dudit signal représentatif de son ($U_K$).

5. Sphygmomanomètre selon la revendication 3, caractérisé en ce que ladite seconde valeur-limite dépend de façon croissante de la pression (p) régnant dans la chambre déformable (3).

6. Sphygmomanomètre selon l'une des revendications 2 à 5, caractérisé en ce que lesdits moyens de détermination de seuil répondent en outre à une action extérieure en sélectionnant une valeur de seuil parmi deux valeurs différentes déterminées chacune en dépendance, de façon croissante, de la valeur dudit signal représentatif de son ($U_K$).

**Patentansprüche**

1. Sphygmomanometer umfassend:
- Eine Manschette (1), die dazu bestimmt ist, an einem Glied eines zu untersuchenden Subjekts befestigt zu werden, wobei diese Manschette eine deformierbare Kammer (3) aufweist;
- Kompressionsmittel (21, 35, 41) zum Erhöhen des Gasdrucks (p), der in der besagten Kammer herrscht;
- Druckminderungsmittel (37) zum Vermindern des besagten Drucks:
- Mittel zur Druckmessung (39), die auf den besagten Druck ansprechen, indem sie ein Drucksignal ($U_p$) liefern, das für diesen Druck repräsentativ ist:
- Mittel zur Tonerfassung (5, 51), die auf jeden Ton reagieren, der in einem Bereich enthaltend die besagte Manschette ausgesandt wird, indem ein Signal ($U_K$) erzeugt wird, das für den besagten Ton repräsentativ ist;
- Mittel (105, 107, 109, 111, 113, 115, 117, 119, 121, 123) zum Bestimmen eines Schwellenwertes ($U_R$), die auf das Drucksignal ($U_p$) ansprechen, indem sie die besagte Schwelle als Funktion der Amplitude des besagten Drucksignals einstellen;
- Mittel zur Bestätigung (53), die auf den besagten Schwellenwert als auch auf die bezüglich des Tons repräsentativen Signale ($U_K$) ansprechen, deren Wert die besagte Schwelle ($U_R$) überschreitet, indem sie ein Bestätigungssignal liefern; und
- Steuermittel (55, 61, 63, 65), die auf die besagten Drucksignale ($U_p$) und Bestätigungssignale ansprechen, indem sie die Amplituden, die das besagte Drucksignal ($U_p$) während des Auftretens des ersten bzw letzten Bestätigungssignals aufweist, messen und speichern;
dadurch gekennzeichnet, daß die besagten Mittel zum Bestimmen der Schwelle die besagte Schwelle ($U_R$) in Abhängigkeit von der Amplitude des Drucksignals ($U_p$) in zunehmender Weise einstellen.

2. Sphygmomanometer gemäß Anspruch 1, dadurch gekennzeichnet, daß die besagten Mittel zum Bestimmen der Schwelle zudem auf jedes für einen aktiven Ton repräsentatives Signal ($U_K$), dessen Wert den Momentanwert der besagten Schwelle überschreitet, ansprechen, indem sie die besagte Schwelle in Abhängigkeit vom Wert dieses Tonsignals in zunehmender Weise regeln.

3. Sphygmomanometer gemäß Anspruch 2, dadurch gekennzeichnet, daß die besagten Mittel zum Bestimmen der Schwelle auf das Anlagen des besagten für einen aktiven Ton repräsentativen Signals ($U_K$) ansprechen, indem sie den Wert der besagten Scnwelle erhöhen, indem sie diesen gemäß einer ersten Zeitkonstante gegen einen ersten Grenzwert gehen lassen, und indem sie beim Abklingen des besagten Signals diesen Schwellenwert vermindern, indem sie diesen gemäß einer zweiten Zeitkonstante unterhalb der ersten Zeitkonstanten gegen einen zweiten Grenzwert gehen lassen.

4. Sphygmomanometer gemäß Anspruch 3, dadurch gekennzeichnet, daß der besagte erste Grenzwert in zunehmender Weise von dem Wert des besagten für den Ton repräsentativen Signals ($U_K$) abhängt, wenn ansonsten alles gleich ist.

5. Sphygmomanometer gemäß Anspruch 3, dadurch gekennzeichnet, daß der besagte zweite Grenzwert von dem Druck (p), der in der deformierbaren Kammer (3) herrscht, in zunehmender Weise abhängt.

6. Sphygmomanometer nach einem der Ansprüche 2 bis 5, dadurch gekennzeichnet, daß die besagten Mittel zum Bestimmen der Schwelle zudem auf eine äußere Einwirkung ansprechen, indem sie einen Schwellenwert unter zwei verschiedenen bestimmten Werten jeweils in Abhängigkeit in zunehmender Weise von dem Wert des besagten für den Ton repräsentativen Signals ($U_K$) auswählen.

8

## Claims

1. A sphygmomanometer comprising:
- a band (1) intended to be secured to a limb of an individual having to be examined, said band having a deformable chamber (3);
- compression means (21, 35, 41) for increasing the gas pressure (p) prevailing in said chamber;
- relief means (37) for reducing said pressure;
- pressure measurement means (36) reacting to said pressure by providing a pressure signal (U) representative of said pressure;
- sound detection means (5, 51) reacting to each sound emitted in
- a zone including said band, by issuing a signal $(U_K)$ representative of said sound:
- threshold value $(U_R)$ setting means (105, 107, 103, 111, 113, 115, 117, 119, 121, 123) that are responsive to the pressure signal $(U_p)$ by adjusting said threshold in dependence on the amplitude of said pressure signal;
- validation means (53) responsive to said threshold value and to the sound representative signals $(U_K)$ having a value greater than said threshold $(U_R)$, by issuing a validation signal; and
- control means (55, 61, 63, 65) responsive to said pressure $(U_p)$ and validation signals, by measuring and memorizing the amplitudes of said pressure signal $(U_p)$ when the first and last validation sigals appear:
characterized in that said threshold setting means adjust said theshold $(U_R)$ in dependence, increasingly, on the amplitude of the pressure signal $(U_p)$.

2. A sphygmomanometer as in claim 1, characterized in that said threshold setting means additionally respond to each active sound-representative signal $(U_K)$ having a value greater than the instantaneous value of said threshold by regulating said threshold in dependence, increasingly, on the value of said sound signal.

3. A sphygmomanometer as in claim 2, characterized in that said threshold setting means respond to the application of said active sound representative signal $(H_K)$ by increasing the value of said threshold by causing it to tend, in accordance with a first time constant, towards a first limiting value and to the disappearance of said signal by decreasing said threshold value by causing it to tend, in accordance with a second time constant less than said first time constant, towards a second limiting value.

4. A sphygmomanometer as in claim 3, characterized in that said first limiting value depends, increasingly, all other things being otherwise equal, on the value of said sound representative signal $(U_K)$.

5. A sphygmomanometer as in claim 3, characterized in that said second limiting value depends, increasingly, on the pressure (p) prevailing in the deformable chamber (3).

6. A sphygmomanometer as in any one of claims 2 to 5, characterized in that said threshold setting means additionally respond to an external action by selecting a threshold value from among a pair of different values each set in dependence, increasingly, on the value of said sound representative signal $(U_K)$.

# Fig.1

# Fig.2

Fig. 3

Fig. 4

Fig. 5

Fig. 6

0 064 475